# EUROPÄISCHE PATENTANMELDUNG

(11) **EP 2 823 854 A1**
(43) Veröffentlichungstag der Anmeldung: **14.01.2015**
(21) Anmeldenummer: 14002155.1
(22) Anmeldetag: 24.06.2014
(51) Int. Cl.: A61N 1/36, A61N 1/04

(54) **Stimulationsvorrichtung**

(30) Priorität: 10.07.2013 DE 102013011530
(71) Anmelder: cerbomed GmbH, 91052 Erlangen (DE)
(72) Erfinder: Zschaeck, Thomas, 90427 Nürnberg (DE); Hartlep, Andreas, 83607 Holzkirchen (DE)
(74) Vertreter: Gosdin, Michael

(57) **Zusammenfassung**

Die Erfindung betrifft eine Stimulationsvorrichtung (1) zur Aufbringung eines transkutanen elektrischen Stimulationsreizes auf die Oberfläche eines Abschnittes des menschlichen Ohrs (2), die ein am oder im Ohr (2) anbringbares Halteelement (3) sowie mindestens zwei Elektroden (4, 5) aufweist, die in oder an einem Elektrodenträger (6) angeordnet sind, wobei die beiden Elektroden (4, 5) am Elektrodenträger (6) durch einen elektrisch isolierenden Abschnitt (7) voneinander getrennt sind. Um eine gute elektrische Leitfähigkeit der Elektroden bei hohem Tragekomfort zu erreichen, sieht die Erfindung vor, dass die mindestens zwei Elektroden (4, 5) von einem Mantelelement (8) umhüllt sind, wobei das Mantelelement (8) mindestens zwei Hüllelemente (9, 10) aufweist, die je einen Aufnahmebereich (12, 13) für je eine Elektrode (4, 5) haben, wobei das Material der Hüllelemente (9, 10) elektrisch leitend oder elektrisch leitfähig machbar ist, wobei das Mantelelement (8) mindestens einen Isolationsabschnitt (11) aus elektrisch isolierendem Material aufweist, der mindestens zwei Hüllelemente (9, 10) miteinander verbindet.

## Beschreibung

Die Erfindung betrifft eine Stimulationsvorrichtung zur Aufbringung eines transkutanen elektrischen Stimulationsreizes auf die Oberfläche eines Abschnittes des menschlichen Ohrs, die ein am oder im Ohr anbringbares Halteelement sowie mindestens zwei Elektroden aufweist, die in oder an einem Elektrodenträger angeordnet sind, wobei die mindestens zwei Elektroden am Elektrodenträger durch einen elektrisch isolierenden Abschnitt voneinander getrennt sind.

Eine gattungsgemäße Stimulationsvorrichtung ist aus der DE 10 2012 014 714 A1 bekannt. Die DE 10 2010 015 277 A1 zeigt eine ähnliche Lösung.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zur invasiven als auch zur non-invasiven Stimulation.

Die vorliegende Erfindung stellt auf die Methode der transkutanen elektrischen Nervenstimulation ab. Bei diesem Verfahren werden Impulsströme verschiedener Stromformen, Amplituden, Impulsdauern und Frequenzen durch die Haut hindurch an verschiedenen Nerven appliziert und verändern deren Statusparameter in vorteilhafter Weise.

Eine Stimulationsvorrichtung der eingangs genannten Art ist aus der DE 10 2010 054 165 B3 bekannt. Hier ist eine Elektrodenanordnung beschrieben, die ein am Ohr anbringbares Halteelement aufweist. An diesem ist über einen elastischen Abschnitt ein Elektrodenträger angeordnet, der zwei Elektroden hält. Der Elektrodenträger besteht aus elektrisch nicht-leitendem Kunststoff und trägt die beiden Elektroden aus Metall.

Wenngleich die vorbekannte Stimulationsvorrichtung bereits zu guten Behandlungsergebnissen führt, haben sich in der Praxis gewisse Nachteile der Anordnung herausgestellt. Hierbei ist die Kontaktqualität der Elektroden zu nennen, die mitunter noch nicht optimal ist, da die von den Elektroden akquirierten Hautareale nicht ausreichend groß sind. Die Erhöhung des Anpressdrucks auf die Hautoberfläche ist zumeist keine geeignete Maßnahme zur Behebung dieser Schwierigkeiten, da hierdurch die Haut des Patienten zu stark belastet wird.

Der Erfindung liegt daher die Aufgabe zugrunde, eine Stimulationsvorrichtung der gattungsgemäßen Art so fortzubilden, dass die genannten Nachteile überwunden werden. Es soll also eine Weiterbildung dahingehend vorgeschlagen werden, dass die Kontaktqualität der Elektroden verbessert wird, ohne die Hautoberfläche des Patienten stark zu belasten, so dass ein hoher Tragekomfort erreichbar ist.

Die Lösung dieser Aufgabe durch die Erfindung ist dadurch gekennzeichnet, dass die mindestens zwei Elektroden der Stimulationsvorrichtung von einem Mantelelement umhüllt sind, wobei das Mantelelement mindestens zwei Hüllelemente aufweist, die je einen Aufnahmebereich, insbesondere eine Aufnahmekammer, für je eine Elektrode haben, wobei das Material der Hüllelemente elektrisch leitend oder elektrisch leitfähig machbar ist, wobei das Mantelelement mindestens einen Isolationsabschnitt aus elektrisch isolierendem Material aufweist, der mindestens zwei Hüllelemente miteinander verbindet.

Die beiden Elektroden erstrecken sich vorzugsweise vom Elektrodenträger in zwei im wesentlichen voneinander weg gerichteten Richtungen, wobei das Mantelelement im wesentlichen länglich ausgebildet ist.

Die Hüllelemente können halbkugelschalenförmig oder halbellipsoidschalenförmig ausgebildet sein. Sie entsprechen der Form der Elektroden, die sie umgeben. Eine Alternative sieht vor, dass die mindestens zwei Hüllelemente hohlzylindrisch ausgebildet sind.

Der Isolationsabschnitt weist bevorzugt eine im wesentlichen zylindrische Form auf. Der Isolationsabschnitt kann dabei in seinem Mittenbereich eine Einschnürung aufweisen. Weiterhin kann das Mantelelement eine Öffnung zum Durchtritt des Elektrodenträgers aufweisen.

Gemäß einer alternativen Ausgestaltung kann vorgesehen sein, dass der Isolationsabschnitt eine im wesentlichen stabförmige Gestalt aufweist, wobei die axialen Enden des Isolationsabschnitts an einer Umfangsstelle der Hüllelemente befestigt sind.

Die Hüllelemente bestehen bevorzugt aus einem offenporigen porösen Material, wobei besonders an ein schwammartiges Material gedacht ist. Dieses Material ist bevorzugt dehnbar, so dass es sich elastisch um die Elektrode legt, die vom Hüllelement umgeben wird.

In diesem Falle ist weiter bevorzugt vorgesehen, dass das Material der Hüllelemente mit einer Flüssigkeit oder mit einem Gel getränkt ist. Dies stellt für die oben erwähnte Maßnahme, das Material der Hüllelemente elektrisch leitfähig zu machen, das Mittel der Wahl dar. Demgemäß ist das Material des Hüllelements von sich aus zwar offenporig, aber nicht elektrisch leitend. Durch Einbringung einer Flüssigkeit oder eines Gels (d. h. eines Elektrolyts) wird die elektrische Leitfähigkeit dann hergestellt.

Die Hüllelemente können auch aus einem geschlossenporigen Material bestehen, das bevorzugt weich und/oder dehnbar ist. Die elektrische Leitfähigkeit kann dann durch andere Maßnahmen hergestellt werden. Hier kommt beispielsweise in Betracht, dem Ausgangsstoff für das Hüllelement Kohlenstoff- oder Metallpartikel zuzumischen, was zur elektrischen Leitfähigkeit der fertig hergestellten Hüllelemente führt.

Das Mantelelement ist bevorzugt als einstückiges Formteil ausgebildet.

Die Elektroden sind bevorzugt Metallelektroden, insbesondere Titanelektroden. Sie weisen bevorzugt die Form eines Kugelabschnitts oder eines Abschnitts eines Ellipsoids auf. Der Elektrodenträger weist, insbesondere als Elektrodenkopf ausgebildet, bevorzugt mindestens eine Stimulationselektrode und mindestens eine Referenzelektrode auf.

Die Teile der Stimulationsvorrichtung bestehen bevorzugt - soweit Hautkontakt gegeben ist - aus einem weichen Material, wobei speziell an ein Elastomermaterial gedacht ist, insbesondere an Silikon oder an ein Material, das Silikon aufweist.

Vorteilhaft ist es, dass durch die vorgeschlagene Ausgestaltung der Stimulationsvorrichtung eine gute Kontaktqualität der Elektroden bei der Auflage auf der Haut gegeben ist, ohne dass es eines hohen Anlagedrucks der Elektroden auf der Haut des Patienten bedarf.

In der Zeichnung sind Ausführungsbeispiele der Erfindung dargestellt. Es zeigen:
- Fig. 1: die Ansicht eines Ohrs mit einer Stimulationsvorrichtung, die in das Ohr eingesetzt ist (hier noch ohne Mantelelement),
- Fig. 2: in perspektivischer Ansicht einen Teil der Stimulationsvorrichtung mit Elektrodenträger und zwei Elektroden, wobei ein Mantelelement am Elektrodenträger angebracht ist,
- Fig. 3: in Explosionsdarstellung die Stimulationsvorrichtung nach Fig. 2, wobei das Mantelelement vom Elektrodenträger entfernt angeordnet ist,
- Fig. 4: einen Schnitt durch das Mantelelement gemäß Fig. 2 bzw. Fig. 3 und
- Fig. 5: in perspektivischer Ansicht ein Mantelelement gemäß einer alternativen Ausgestaltung der Erfindung.

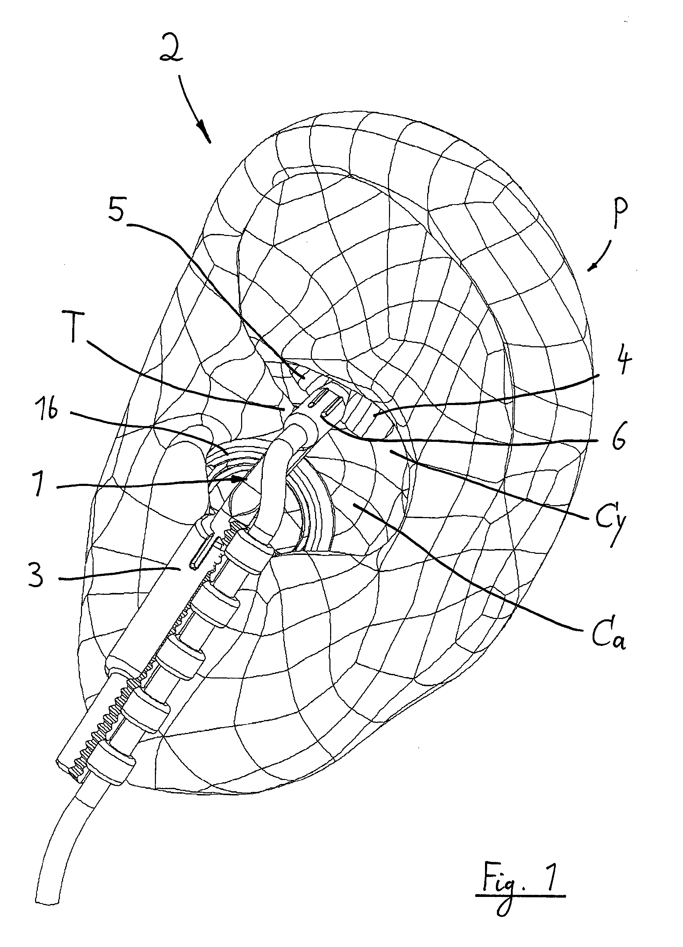

In Fig. 1 ist ein Ohr 2 eines Menschen dargestellt, in das eine Stimulationsvorrichtung 1 eingesetzt ist, um eine transkutane elektrische Stimulation vornehmen zu können. Hiermit soll bevorzugt der Vagusnerv einer Elektrostimulation unterzogen werden, um verschiedenartige Krankheiten zu behandeln bzw. Effekte zu erzielen. Mit der Stimulationsvorrichtung 1 kann demgemäß konkret auf einen Oberflächenbereich des Ohres eine transkutane elektrische Nervenstimulation vorgenommen werden. Hierfür weist die Elektrodenanordnung eine Stimulationselektrode und eine Referenzelektrode (s. unten) auf, zwischen denen ein elektrisches Potential erzeugt wird; die hierfür nötigen Mittel sind im Stand der Technik hinlänglich bekannt, so dass sie hier nicht weiter beschrieben werden müssen. Exemplarisch wird auf die DE 10 2005 003 735 B4 der Anmelderin verwiesen und hierauf ausdrücklich Bezug genommen. Das Mantelelement (s. unten) ist zur Verdeutlichung in Fig. 1 noch nicht dargestellt.

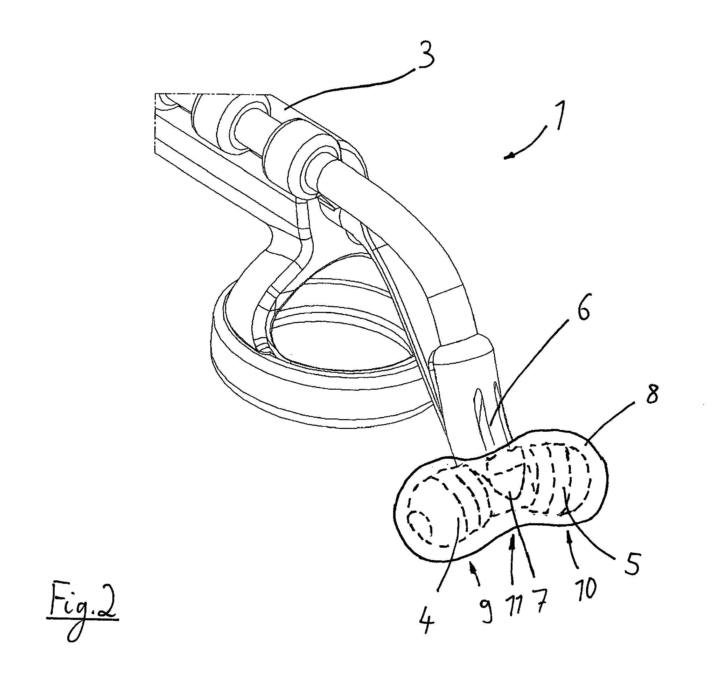

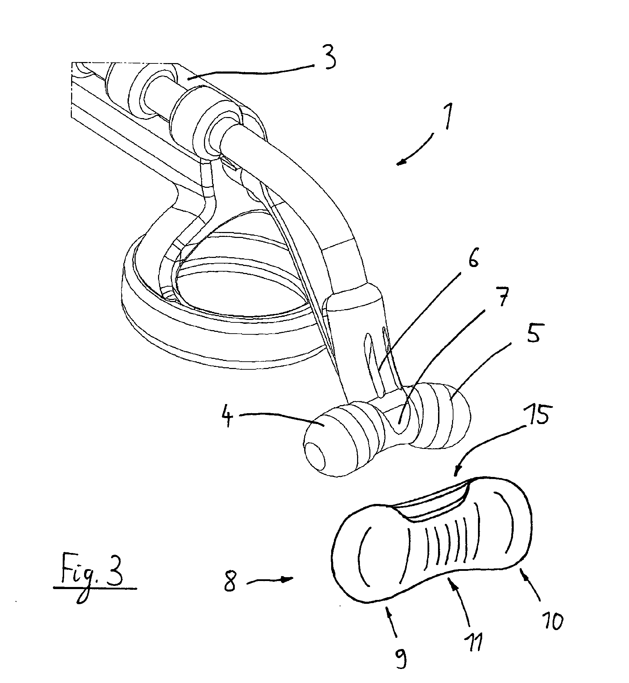

Die Stimulationsvorrichtung weist - wie es im weiteren in den Figuren 2 und 3 zu sehen ist - ein Halteelement 3 auf, das einen Elektrodenträger 6 trägt. Am Elektrodenträger 6 sind zwei Elektroden 4 und 5 angeordnet, wobei die Elektrode 4 eine Stimulationselektrode und die Elektrode 5 eine Referenzelektrode ist. Die beiden Elektroden 4, 5 werden durch einen elektrisch isolierenden Abschnitt 7 des Elektrodenträgers 6 voneinander getrennt, um die Potentialdifferenz zwischen den Elektroden 4, 5 aufbauen zu können. Zum Aufbau der Stimulationsvorrichtung wird ausdrücklich auf die DE 10 2010 054 165 B3 der Anmelderin Bezug genommen, wo diese Stimulationsvorrichtung im Detail beschrieben ist.

In Fig. 1 ist die Pinna P des Ohrs 2 zu sehen. Der Elektrodenträger 6 kommt im Bereich der Cymba conchae Cy zu liegen; indes liegt die Stimulationsvorrichtung 1 mit einem ringförmigen Auflageteil 16 im Bereich des Cavum conchae Ca auf. Demgemäß kommt das ringförmige Auflageteil 16 unterhalb des Tragus T zu liegen.

Wesentlich ist nun, dass die beiden Elektroden 4 und 5 von einem Mantelelement 8 umhüllt sind, wie es in den Figuren 2, 3 und 4 zu erkennen ist. Das Mantelelement 8 weist im Ausführungsbeispiel zwei Hüllelemente 9 und 10 auf, die je einen Aufnahmebereich in Form einer Aufnahmekammer 12 bzw. 13 für je eine Elektrode 4 bzw. 5 haben. Das Material der beiden Hüllelemente 9 und 10 ist hier elektrisch leitfähig gemacht. Dies erfolgte hier so, dass auf ein offenporiges Basismaterial für die Hüllelemente 9 und 10 zurückgegriffen wurde, wobei ansonsten keine Maßnahmen ergriffen wurden, um das aus Kunststoff bestehende Material elektrisch leitfähig zu machen. Dann wurden zur Herstellung besagter Leitfähigkeit die Hüllelemente 9, 10 in einem Gel getränkt, das in die offenporige Struktur des Materials der Hüllelemente 9, 10 eingedrungen ist und so die gewünschte und benötigte elektrische Leitfähigkeit herstellte.

Das Mantelelement 8 weist weiterhin einen Isolationsabschnitt 11 aus elektrisch isolierendem Material auf; dieser Isolationsabschnitt 11 verbindet die beiden Hüllelemente 9 und 10 miteinander und isoliert so auch die an den Elektroden 5, 6 anliegende Potentialdifferenz.

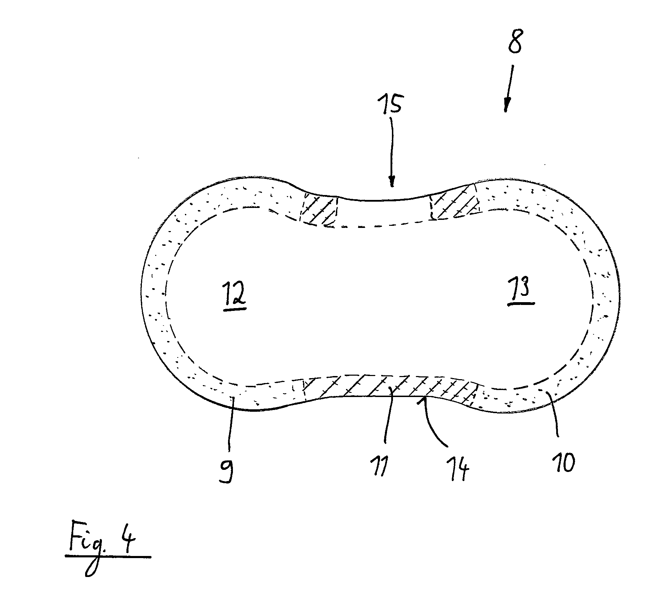

Wie es am besten aus Fig. 4 hervorgeht, haben die beiden Hüllelemente 9, 10 jeweilige Aufnahmekammern 12 und 13, die der Form der Elektroden 4, 5 angepasst sind. Dabei sind das Mantelelement 8 und namentlich die Hüllelemente 9, 10 etwas kleiner gefertigt, als es der Form der Elektroden 4, 5 entspricht. Dies erlaubt es, dass das Mantelelement 8 beim Aufziehen auf den Elektrodenträger 6 samt Elektroden 4, 5 mit leichter elastischer Vorspannung anliegt, so dass die Oberfläche der Elektroden 4, 5 an die innere Oberfläche der Hüllelemente 9, 10 gedrückt wird. Eine gute Anlage des Mantelelements 8 am Elektrodenträger 6 wird ferner durch eine Einschnürung 14 begünstigt, die der ansonsten im wesentlichen hohlzylindrisch ausgebildete Isolationsabschnitt 11 aufweist.

Zum Einführen des Elektrodenträgers samt Elektroden weist das Mantelelement 8 eine Öffnung 15 auf. Die Elastizität des Materials des Mantelelements 8 erlaubt die Anlage desselben um den Elektrodenträger 6 und die Elektroden 4, 5 herum bei relativ geringfügiger Größe der Öffnung 15.

Vorliegend erstecken sich die beiden Elektroden 4 und 5 in voneinander weg gerichtete Richtungen vom Elektrodenträger 6 weg. Das Mantelelement 8 ist demgemäß - wie es am besten in Fig. 4 zu sehen ist - als sich längserstreckendes Teil ausgeführt.

Das Mantelelement 8 kann - wie auch aus Fig. 4 hervorgeht - als einstückiges Teil gefertigt werden, wobei unterschiedliche Materialien einmal für die Hüllelemente 9, 10 und einmal für den Isolationsabschnitt 11 zum Einsatz kommen.

Möglich wäre es auch noch, das Mantelelement durch weitere Befestigungsmittel am Elektrodenträger festzulegen. Hierzu kann an ein Gummiband gedacht werden, das das Mantelelement entlang des Umfangs der Öffnung 15 an den Elektrodenträger 6 spannt.

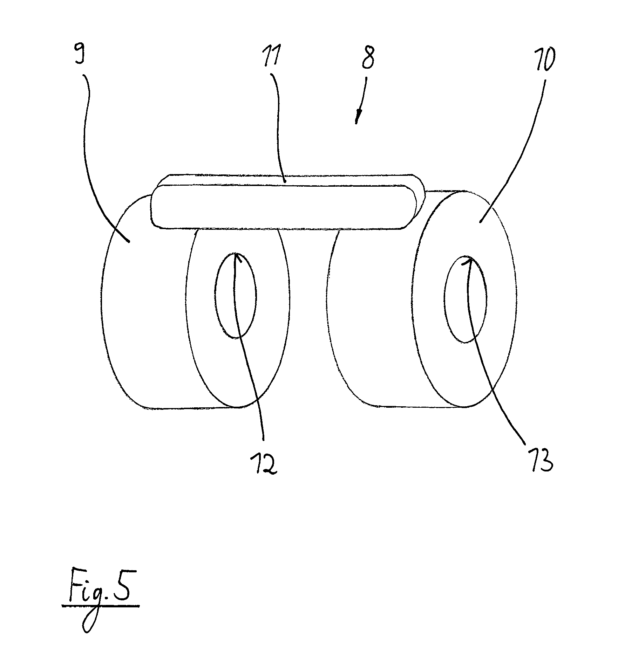

In Fig. 5 ist eine alternative Gestaltung des Mantelelements 8 zu sehen, das hier als eine Art Ummantelung für die Elektroden 4, 5 ausgeführt ist. Es sind wiederum die beiden Hüllelemente 9 und 10 vorgesehen, die hier hohlzylindrisch ausgebildet sind. Das Mantelelement 8 wird hergestellt durch die Anbringung (z. B. Verklebung oder Verschweißung) des Isolationsabschnitts 11, der hier stabförmig ausgeführt ist. Hierdurch werden die beiden Hüllelemente 9, 10 gegeneinander elektrisch isoliert auf definiertem Abstand gehalten. Die Elektroden 4, 5 werden in die zylindrischen Aufnahmebereiche 12 und 13 eingeschoben. Wiederum gilt, dass die Hüllelemente 9, 10 elektrisch leitfähig sein müssen, während der Isolationsabschnitt 11 elektrisch isolierend sein muss.

Eine Anzahl von Mantelelementen 8 kann auch in einem Spender bevorratet werden. Der Spender kann eine entsprechende Anzahl an Vorratstaschen haben, die durch eine Plastikfolie flüssigkeitsdicht abgeschossen sind. Je ein Mantelelement 8 kann in einer entsprechenden Vorratstasche untergebracht werden. Wird ein neues Mantelelement 8 benötigt, wird die Vorratstasche aufgerissen und das Mantelelement entnommen.

Dabei kann bevorzugt vorgesehen werden, dass in der flüssigkeitsdicht abgeschlossenen Vorratstasche bereits ein geeignetes Elektrolyt eingebracht ist, so dass die Hüllelemente des Mantelelements in einem gewünschten, d. h. elektrisch leitfähigen Zustand gehalten werden, in dem das Mantelelement 8 bis zu seinem Einsatz unter optimalen Bedingungen für die transkutane Stimulation gehalten wird.

### Bezugszeichenliste:

- 1: Stimulationsvorrichtung
- 2: Ohr
- 3: Halteelement
- 4: Elektrode (Stimulationselektrode)
- 5: Elektrode (Referenzelektrode)
- 6: Elektrodenträger
- 7: elektrisch isolierender Abschnitt
- 8: Mantelelement (Ummantelung)
- 9: Hüllelement
- 10: Hüllelement
- 11: Isolationsabschnitt
- 12: Aufnahmebereich (Aufnahmekammer)
- 13: Aufnahmebereich (Aufnahmekammer)
- 14: Einschnürung
- 15: Öffnung
- 16: ringförmiges Auflageteil

- Ca: Cavum conchae
- Cy: Cymba conchae
- T: Tragus
- P: Pinna

## Patentansprüche

1. Stimulationsvorrichtung (1) zur Aufbringung eines transkutanen elektrischen Stimulationsreizes auf die Oberfläche eines Abschnittes des menschlichen Ohrs (2), die ein am oder im Ohr (2) anbringbares Halteelement (3) sowie mindestens zwei Elektroden (4, 5) aufweist, die in oder an einem Elektrodenträger (6) angeordnet sind, wobei die mindestens zwei Elektroden (4, 5) am Elektrodenträger (6) durch einen elektrisch isolierenden Abschnitt (7) voneinander getrennt sind,
**dadurch gekennzeichnet,**
**dass** die mindestens zwei Elektroden (4, 5) von einem Mantelelement (8) umhüllt sind, wobei das Mantelelement (8) mindestens zwei Hüllelemente (9, 10) aufweist, die je einen Aufnahmebereich (12, 13) für je eine Elektrode (4, 5) haben, wobei das Material der Hüllelemente (9, 10) elektrisch leitend oder elektrisch leitfähig machbar ist, wobei das Mantelelement (8) mindestens einen Isolationsabschnitt (11) aus elektrisch isolierendem Material aufweist, der mindestens zwei Hüllelemente (9, 10) miteinander verbindet.

2. Stimulationsvorrichtung nach Anspruch 1, **dadurch gekennzeichnet, dass** sich die mindestens zwei Elektroden (4, 5) vom Elektrodenträger (6) in zwei im wesentlichen voneinander weg gerichteten Richtungen erstrecken, wobei das Mantelelement (8) im wesentlichen länglich ausgebildet ist.

3. Stimulationsvorrichtung nach Anspruch 1 oder 2, **dadurch gekennzeichnet, dass** die mindestens zwei Hüllelemente (9, 10) halbkugelschalenförmig oder halbellipsoidschalenförmig ausgebildet sind.

4. Stimulationsvorrichtung nach Anspruch 1 oder 2, **dadurch gekennzeichnet, dass** die mindestens zwei Hüllelemente (9, 10) hohlzylindrisch ausgebildet sind.

5. Stimulationsvorrichtung nach einem der Ansprüche 1 bis 4, **dadurch gekennzeichnet, dass** der Isolationsabschnitt (11) eine im wesentlichen zylindrische Form aufweist.

6. Stimulationsvorrichtung nach Anspruch 5, **dadurch gekennzeichnet, dass** der Isolationsabschnitt (11) in seinem Mittenbereich eine Einschnürung (14) aufweist.

7. Stimulationsvorrichtung nach einem der Ansprüche 1 bis 4, **dadurch gekennzeichnet, dass** der Isolationsabschnitt (11) eine im wesentlichen stabförmige Gestalt aufweist, wobei die axialen Enden des Isolationsabschnitts (11) an einer Umfangsstelle der Hüllelemente (9, 10) befestigt sind.

8. Stimulationsvorrichtung nach einem der Ansprüche 1 bis 6, **dadurch gekennzeichnet, dass** das Mantelelement (8) eine Öffnung (15) zum Durchtritt des Elektrodenträgers (6) aufweist.

9. Stimulationsvorrichtung nach einem der Ansprüche 1 bis 8, **dadurch gekennzeichnet, dass** die Hüllelemente (9, 10) aus einem offenporigen porösen Material, insbesondere aus einem schwammartigen Material, bestehen, das bevorzugt weich und/oder dehnbar ist.

10. Stimulationsvorrichtung nach Anspruch 9, **dadurch gekennzeichnet, dass** das Material der Hüllelemente (9, 10) mit einer Flüssigkeit oder mit einem Gel getränkt ist.

11. Stimulationsvorrichtung nach einem der Ansprüche 1 bis 8, **dadurch gekennzeichnet, dass** die Hüllelemente (9, 10) aus einem geschlossenporigen Material bestehen, das bevorzugt weich und/oder dehnbar ist.

12. Stimulationsvorrichtung nach einem der Ansprüche 1 bis 11, **dadurch gekennzeichnet, dass** das Mantelelement (8) als einstückiges Formteil ausgebildet ist.
